# EUROPEAN PATENT APPLICATION

(11) **EP 4 778 525 A2**
(43) Date of publication of application: **22.07.2026**
(21) Application number: 26174984.0
(22) Date of filing: 28.04.2026
(51) Int. Cl.: A61K 31/56, A61K 31/575, A61K 8/63, A61Q 19/08

(54) **THE APPLICATION OF GANODERONE A IN THE MANUFACTURE OF CD38 INHIBITORS AND RELATED PRODUCTS**

(30) Priority: 09.02.2026 CN 202610186018
(71) Applicant: Infinitus (China) Company Ltd., Jiangmen, Guangdong 529156 (CN)
(72) Inventor: ZHOU, Zhuang, Guangdong, 529156 (CN); TAI, Meiling, Guangdong (CN)
(74) Representative: Yamankaradeniz, Kemal

(57) **Abstract**

Provided in the present application is an application of Ganoderone A in manufacturing a CD38 inhibitor and related products, comprising applying Ganoderone A to manufacturing a CD38 inhibitor. The present application can solve the problem in the prior art that a CD38 inhibitor has limitations, and is applicable to the field of biochemistry.

## Description

### Technical Field

The present application relates to the field of biochemistry, and in particular, to a use of Ganoderone A in manufacturing a CD38 inhibitor and related products.

### Background

In the field of life sciences and medical applications, research into anti-aging mechanisms and the development of efficient interventional strategies remains at the forefront of scientific inquiry. This is particularly pertinent as population aging accelerates and consumers demonstrate growing demand for skincare products that can delay aging and restore a youthful, vibrant appearance. However, anti-aging products and technologies currently on the market face many challenges, particularly regarding the key factors for aging-namely, the inhibition of CD38 protein activity for delaying skin aging, where existing solutions fall short of optimal efficacy and safety standards.

Anti-CD38 antibodies are recognized for their specific binding to CD38 proteins, yet they possess inherent limitations. The large molecular size of monoclonal antibodies (>150 kDa) hinders penetration through the skin's natural barrier. Even with advanced permeation enhancement technologies, their permeation rate through the stratum corneum remains below 0.1%, constraining their potential as an anti-aging active in skincare. Moreover, high production costs render large-scale production economically unfeasible and limit widespread adoption in the cosmetics industry. Additionally, these antibodies are thermolabile, exhibiting susceptibility to denaturation and activity loss at ambient temperatures, thereby necessitating cold chain logistics. These requirements further increase consumer costs, reduce ease of use, and limit their application in daily skincare products.

On the other hand, small-molecule CD38 inhibitors, such as those based on pyrimidine derivatives or imidazopyridine compounds, although exhibiting high efficiency in inhibiting enzymatic activity, pose risks of off-target cellular effects and non-specific binding. These inhibitors may interfere with other members of the NADase family, disrupt normal metabolic homeostasis, and even activate aberrant signaling pathways. Long-term use may result in serious systemic side effects, including metabolic disorders and increased disease susceptibility. Particularly in topical application, should active ingredients penetrate the bloodstream, they may cause irreversible damage to vital organs such as the liver and kidneys, thereby compromising overall health through cumulative effects.

In summary, prior art approaches to developing CD38 inhibitors for skin anti-aging fail to achieve a balanced combination of efficacy, safety, and practicality, regardless of whether the inhibitor is an antibody or a synthetic small molecule..

### Summary

The main purpose of the present application is to provide the use of Ganoderone A in the manufacture of a CD38 inhibitors and related products, thereby overcoming the limitations inherent in prior art CD38 inhibitors.

In order to achieve the described object, according to the first aspect of the present application, a use of Ganoderone A in manufacturing a CD38 inhibitor is provided.

Furthermore, the concentration of Ganoderone A in the CD38 inhibitor is 50-100 µM.

In order to achieve the above object, according to the second aspect of the present application, an application of Ganoderone A in manufacture of a product resistant to aging is provided.

Further, the product includes a cosmetic, a skin care product, a health care product, or a pharmaceutical product.

Further, anti-aging includes increasing the level of NAD⁺ in the cell and/or decreasing the activity of superoxide in the mitochondria of the cell by inhibiting CD38 protein.

Further, the concentration of Ganoderone A in the product is 50-100µM.

Further, auxiliaries and/or carriers are also included in the product.

In order to achieve the described object, according to the third aspect of the present application, an application of Ganoderone A in manufacture of a product for increasing the level of NAD⁺ in cells or decreasing the activity of superoxide in mitochondria of cells is provided.

Further, the concentration of Ganoderone A in the product is 50-100µM.

Further, auxiliaries and/or carriers are also included in the product.

The present application provides the application of Ganoderone A in the manufacture of a CD38 inhibitor. This application achieves effective CD38 inhibition while leveraging the compound's natural origin, mildness, and safety profile. Accordingly, the present application addresses the deficiencies of both antibody-based and synthetic small-molecule inhibitors described in the prior art, offering an optimal balance between efficacy and biological safety.

### Brief Description of the Drawings

The accompanying drawings, which form a part of the present application, are used to provide a further understanding of the present application. The schematic embodiments of the present application and the description thereof are used to explain the present application, and do not form improper limits to the present application. In the drawings:
Fig. 1 shows the results of testing Ganoderone A for inhibiting CD38 activity in Example 1 of the description of the present application.
Fig. 2 shows a diagram illustrating results of interaction test between Ganoderone A and CD38 in Example 2 of the description of the present application.
Fig. 3 shows a test result diagram of decreasing mitochondrial superoxide of Ganoderone A and CD38 Ganoderone A in embodiment 3 of the description of the present application, wherein A in Fig. 3 is a flow diagram of mitochondrial superoxide, and B in Fig. 3 is a statistical chart of mean fluorescence intensity of various groups of MitoROS.
Fig. 4 shows the results of safety tests of Ganoderone A on skin fibroblasts BJ in Example 4 of the description of the present application.

### Detailed Description of the Embodiments

It is important to note that the embodiments of the present application and the characteristics in the embodiments can be combined under the condition of no conflicts. Hereinafter, the present application will be described in detail with reference to examples.

As mentioned in the background art, the CD38 inhibitor in the prior art has limitations, and when it is applied to antisensibility, it is difficult to achieve effective and safe effects, and the manufacture costs of the antibody-based CD38 inhibitor and the synthetic small-molecule CD38 inhibitor in the prior art are high, with the problems of low bioavailability and potential toxicity side effects. Based on this, the inventor of the present application tries to develop a new CD38 inhibitor, and therefore proposes a series of protection solutions of the present application.

In the first typical embodiment of the present application, the use of Ganoderone A in the manufacture of a CD38 inhibitor is provided.

CD38 (Cluster of differentiation 38) is a widely expressed transmembrane sugar hydrolase, that participates in energy metabolism, cell adhesion and immune response, and is associated with various diseases such as Parkinson's disease, obesity, diabetes, heart disease, ovarian cancer and leukemia. Most CD38 has a dimorphic membrane localization, with the catalytic site facing the extracellular side. More than 90% of CD38 functions as an extracellular NADase, converting NAD⁺ into NAM (nicotinamide) and ADPR (adenosine diphosphate ribose) using β-NAD and its derivatives β-NADP and β-NMN as substrates. Previous reports have indicated that CD38 expression and activity increases with age, leading to NAD⁺ levels and mitochondrial dysfunction during senescence.CD38 is a major enzyme that consumes NAD⁺, and its decreasesd level indicates a key role in regulating NAD⁺ levels in senescence and metabolic diseases.

Ganoderone A is a lanostane-type triterpene compound extracted from Ganoderma lucidum. The formula is shown as below:

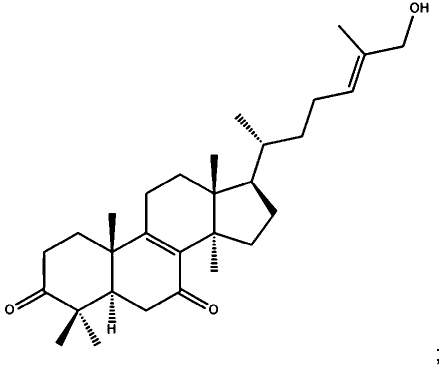

The structural feature of Ganoderone A has a tetracyclic triterpenoid skeleton (a lanostane core) with functional groups such as ketone groups (C=O), hydroxyl groups (-OH), methyl groups (-CH₃) and double bonds. In terms of physicochemical properties, it is usually presented as a light yellow or white crystalline powder, and exhibited strong lipid solubility (poorly soluble in water, easily soluble in organic solvents such as methanol, ethanol and chloroform).The Ganoderone A has a specific melting point (usually in the range of 160-200°C, a nd the specific value varies according to isomers or purity), and may have optical activity.

The inventors of the present application surprisingly found that Ganoderone A exhibits CD38-inhibitory activity. Ganoderone A specifically binds to the CD38 protein, thereby inhibiting its enzymatic activity., thereby preserving NAD⁺ (nicotinamide adenine dinucleotide) levels and achieving anti-ageing effects. The present application reveals that molecular docking analysis demonstrated a binding energy of -8.7 kcal/mol between Ganoderone A and CD38, indicating favorable binding affinity. This threshold is established in the prior art (see Trott, O., & Olson, A. J. (2010). AutoDock Vina: improving the speed and accuracy of docking with a new scoring function, efficient optimization and multithreading. Journal of computational chemistry, 31(2), 455-461.https://pubmed.ncbi.nlm.nih.gov/19499576/), and it is recognized that docking results with binding energies more negative than -5.0 kcal/mol are considered theoretically vsild.. There is a possible high affinity interaction between the surface small molecule and the target protein, and this threshold is often used for preliminary screening of compounds with potential biological activity. It is one of the key indexes for evaluating the reliability of a docking prediction result of a molecule. Therefore, it can be seen that the binding of Ganoderone A to the CD38 protein in the present application is much less than -5.0 kcal/mol, indicating good binding capacity.

The specific embodiments of the present application demonstrate that Ganoderone A can effectively inhibit the activity of the CD38 protein. Through highly specific binding to the CD38 active site, Ganoderone A directly obstructs the enzyme's catalytic function,, thereby preventing NAD⁺ degradation. This effectively maintains the level of NAD⁺ within a cell, anddelays cellular senescence and improves cell function. Moreover, Ganoderone A demonstrates superior biocompatibility compared to synthetic small-molecule inhibitors, without eliciting cytotoxicity or immune responses. Accodingly, the CD38 inhibitor of the present application offers efficious and safe anti-aging properties, enabling the development of improved anti-aging compositions.

In a preferred embodiment, the concentration of Ganoderone A in the CD38 inhibitor is 50-100µM including but not limited to 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100µM.

Maintaining Ganoderone A within the specified concentration range allows it to retain activity while exhibiting optimal biocompatibility and transdermal permeability. In practice, persons skilled in the art may make suitable adjustments within said range based on target product characteristics, including pH, formulation matrix, and storage conditions, as well as desired efficacy, such as the intensity and duration of anti-aging benefits. These optimizations serve to balance pharmacological activity and cosmetic acceptability, ensuring product safety.

In the second typical embodiment of the present application, an application of Ganoderone A in manufacture of a product resistant to aging is provided.

Ganoderone A is a lanostane-type triterpene compound isolated and extracted from Ganoderma lucidum, and has a capability of inhibiting the activity of CD38 protein. As a key metabolic enzyme, the CD38 protein is involved in regulating and controlling the metabolism of NAD⁺, and the level of NAD⁺ is closely related to the cell senescence process. The present application discloses the potential of Ganoderone A as a CD38 inhibitor. It is verified by the specific embodiments of the present application that Ganoderone A can significantly inhibit the enzymatic activity of CD38, thereby reducing NAD⁺ consumption, maintaining intracellular NAD⁺ levels, and improving mitochondrial function and delaying cell senescence processes. This finding not only provides scientific basis for the pharmacological effects of Ganoderone A, but also opens up new approaches for the development of anti-aging products.

Ganoderone A can be used for manufacturing an anti-aging product. As an active ingredient in the product, it can directly interact with the CD38 protein, inhibit the activity of CD38 in degrading NAD⁺, and improve mitochondrial function, thereby achieving effects of anti-aging and treating aging-related diseases.

In a preferred embodiment, the product comprises a cosmetic, dermocosmetic, nutraceutical or pharmaceutical product.

The dosage form of the described products is not particularly restricted. A person skilled in the art would be able to select a suitable forms based on the product specifucaions and prepare corresponding compositions with conventional methods without limation herein..

The dosage forms of the above cosmetic and dermatological manufacture include, but are not limited to, creams, ointments, emulsions, gels, oils, powders, aerosols, film manufacture, and lyophilized manufacture. Dosage forms for such health supplements or medicines include, but are not limited to, solid or liquid formulations; wherein the solid manufacture comprise tablets, capsules, pills, granules, and dispersible powders. Liquid formulations include solutions, sprays, injections, and lotions. A person skilled in the art is able to flexibly select and prepare a corresponding medicine according to the requirements of a product to be produced.

In a preferred embodiment, the anti-aging comprises increasing the level of NAD⁺ in the cell and/or decreasing the activity of superoxide in the mitochondria of the cell by inhibiting CD38 protein.

The coenzyme I in cells namely nicotinamide adenine dinucleotide (NAD⁺), is a key metabolite that maintains normal cellular functions, including energy metabolism, DNA repair, and gene expression regulation. It also acts as a bridge between senescence and various physiological and pathological processes. NAD⁺ is commonly found in organisms in both oxidized form (NAD⁺) and reduced form (NADH), and these two states can interconvert during a series of biological processes within the cell. NAD+ is not only an essential redox cofactor in mitochondria but also a key signaling molecule that regulates cell functions when environmental changes occur, such as fluctuations in nutrient intake or cell damage. These functions encompass mitochondrial function, DNA damage repair, apoptosis, cellular senescence, and signal transduction. Increasing evidence suggests that abnormal NAD⁺ metabolism in cells can affect the structure and function of mitochondria, potentially leading to a variety of diseases.

Numerous studies in the prior art show that in various organ systems, such as the nervous system, the cardiovascular system, the immune system, and the skin, CD38-mediated NAD⁺ degradation is closely associated with the development of age-related diseases. For example, in the nervous system, a decrease in NAD⁺ levels may affect the energy supply and signal conduction of neurons, and accelerate the degeneration of cognitive functions. In the cardiovascular field, NAD⁺ may be involved in vascular endothelial dysfunction and the formation of atherosclerosis. As for the skin, a decrease in NAD⁺ presents obvious signs of aging, such as decreased skin elasticity, increased wrinkles, and pigmentation.

In addition, CD38 influences mitochondrial function by reducing NAD⁺ levels. NAD⁺ is a key electron carrier in mitochondrial oxidative phosphorylation, responsible for transmitting hydrogen ions and electrons through the tricarboxylic acid cycle (TCA cycle) and the electron transport chain to drive ATP synthesis. When CD38 activity is abnormally upregulated, the cytoplasmic NAD⁺ pool becomes depleted, disrupting NAD⁺ homeostasis. As the mitochondrial membrane is impermeable to NAD⁺, its internal levels depend on specific transporters (e.g., SLC25A51) and the salvage synthesis pathway. Depletion of cytoplasmic NAD+ indirectly results in a decreased availability of NAD⁺ within the mitochondrial matrix. This directly impairs the activity of NAD+-dependent dehydrogenases, thereby reducing the flux of the TCA cycle and the supply of reducing equivalents (NADH/FADH2). Ultimately, this leads to diminished ATP production and a cellular energy crisis.

Moreover, NAD⁺ is an essential cofactor for sirtuins (class III histone deacetylases). SIRT3, the primary mitochondrial deacetylase, requires NAD⁺ for catalytic activity. SIRT3 regulates various mitochondrial metabolism and antioxidant proteins, including complex I, acetyl-CoA synthetase 2, and superoxide dismutase 2, through deacetylation modifications. CD38-mediated NAD⁺ depletion will directly inhibit the activity of SIRT3, leading to excessive acetylation of mitochondrial proteins, thereby causing a decrease in electron transport chain efficiency, excessive generation of reactive oxygen species, and damage to the antioxidant defense system.

The resulting metabolic dysfunction and oxidative stress not only impair mitochondrial bioenergetics but also induce mitochondrial membrane potential collapse, permeability transition pore opening, and apoptosis.

The present application provides a CD38 inhibitor that contains Ganoderone A, which can increase the level of NAD⁺ (nicotinamide adenine dinucleotide) in cells and/or decrease superoxide activity in cellular mitochondria, thereby achieving the objectives of optimizing cell function and delaying the progression of senescence. In the specific embodiments of this application, the key active ingredient, Ganoderone A, precisely targets the CD38 protein, effectively regulating the metabolic balance of NAD⁺ at the cellular level. Ganoderone A directly enhances the available amount of NAD⁺ in cells by inhibiting the CD38 enzyme from degrading NAD⁺, thus providing more sufficient energy for cells, enhancing the DNA repair mechanism, delaying cellular senescence, and improving their health status and functional performance. Furthermore, Ganoderone A not only directly inhibits the CD38 protein but also indirectly enhances mitochondrial function by maintaining NAD⁺ levels, thereby reducing reactive oxygen species (ROS) generation, decreasing superoxide activity, and protecting mitochondria from oxidative damage, which further delays the senescence process of cells and tissues.

The Ganoderone A mentioned above, as applied in this context, has the ability to inhibit the CD38 protein. By doing so, it optimizes NAD⁺ metabolism, reduces mitochondrial reactive oxygen species (ROS) generation, and improves cellular energy efficiency. Additionally, Ganoderone A strengthens DNA repair capacity, enhances the cell's antioxidant functions, and mitigates oxidative stress-induced cell damage. Consequently, cellular viability is improved, senescence is delayed, and a theoretical foundation and practical technical solution are provided for the field of senescence prevention..

In a preferred embodiment, the concentration of Ganoderone A in the product is 50-100µM.

In a preferred embodiment, adjuvants and/or carriers are included in the product.

In addition to containing the described Ganoderone A, the described product may also optionally contain auxiliaries commonly used in the manufacture of cosmetics, skin care products, health care products, or medicines, as well as pharmaceutically acceptable auxiliaries and/or carriers. Such adjuvants include, but are not limited to, antioxidants (e.g., vitamin E, green tea extract), humectants (e.g., hyaluronic acid, glycerol), collagen promoters (e.g., peptidic compounds), and other pharmaceutically acceptable adjuvants (e.g., solvents, stabilizers, diluents, binders, antisticking agents, cosolvents, film coating materials, slow-release matrix materials, solubilizers, emulsifiers, preservatives, isotonic modulators, colorants, etc.). A person skilled in the art may flexibly select an auxiliary material and/or carrier according to actual needs, and this selection is not limited in the present application.

In the third typical embodiment of the present application, an application of Ganoderone A in increasing the level of NAD⁺ in cells is provided.

In a preferred embodiment, the concentration of Ganoderone A in the product is 50-100µM.

In a preferred embodiment, adjuvants and/or carriers are included in the product.

In a fourth typical embodiment of the present application, the use of Ganoderone A in reducing the activity of superoxide in the mitochondria of cells is provided.

In a preferred embodiment, the concentration of Ganoderone A in the product is 50-100µM.

In a preferred embodiment, adjuvants and/or carriers are included in the product.

The beneficial effects of the present application will be further explained in detail below in conjunction with specific embodiments.

The experimental materials, experimental instruments and reagent information used in the embodiments of the present application are as follows (except for the specific description, the reagents in the embodiments of the present application are all conventional and commercially available products).
1. Experimental Materials:
   (1) 293t cells purchased from the China Center for Type Culture Collection.
   (2) C57 mice purchased from Jiangsu Pharmaceutica Biotechnology Co., Ltd.
2. Experimental instrument:
   (1) Multifunction microplate reader, model Synergy H1, available from Boitek.
   (2) BioX-Ray Irradiators, Model RS 2000Plus, purchased from Rad Source Technologies, Inc.
3. Main reagents:
   (1) Ganoderone A, cat. no. B50043, purchased from a source foliar organism.
   (2) High glucose DMEM medium available from Gibco under the designation C11995500BT.
   (3) PBS pH = 7.4, grade C10010500BT, purchased from Gibco.
   (4) NAD⁺ standard, CAS 53-84-9, purchased from Roche.
   (5) Cell Counting kit 8 (CCK8), Catalogue C0040, purchased from Shangdong City.
   (6) Biglycin, CAS 556-50-3, purchased from Aladine.
   (7) Ethanol dehydrogenase, CAS 9031-72-5, purchased from Sigma-Aldrich.
   (8) Nicotinamide, CAS 98-92-0.
   (9) Anhydrous ethanol, CAS 64-17-5, was purchased from Guangzhou brand.
   (10) 1,1,2-trichlorotrifluoroethane CAS 76-13-1, purchased from Nanjing Chemicals Co., Ltd.
   (11) Trioctylamine, Cat. No. T818834, from McLinin.
4. Solution manufacture:
   (1) manufacturing an NAD⁺ reaction solution, wherein a 1 mL reaction solution system: 61 mM biglycinin, 820 µL; Cell Counting kit8, 100 µL; 2.6 KU/mL Ethanol dehydrogenase, 5 µL; 3.5 M Nicotinamide, 28.5 µL; absolute ethanol, 57 µL.
   (2) Manufacture of an NAD⁺ extract: 1,1,2-trichlorotrifluoroethane and trioctylamine are prepared at a volume ratio of 3:1, mixed and vortexed, and now used.

### Example 1: Test for inhibition of CD38 activity by Ganoderone A

1. The 293T-CD38 was used to degrade NAD⁺: in a 96 well plate, 3 wells of cells were assayed and a solution of NAD⁺ in PBS containing a concentration of 1µM was added in a total volume of 150µL as a blank control (mock); 1×10⁴ 293T-CD38 cells per well of the other three wells; after being attached to the walls of the cells, a PBS solution containing NAD⁺ at a concentration of 1µM is added, the total volume being 150 µL; as a control, 1×10⁴ 293T-CD38 cells per well of the other wells are added; after being attached to the walls of the cells, a PBS solution containing NAD⁺ at a concentration of 1µM is added respectively; and Ganoderone A added to the solution has final concentrations of 2.5, 1.25, 0.625 and 0µM, the total volume being 150 µL.

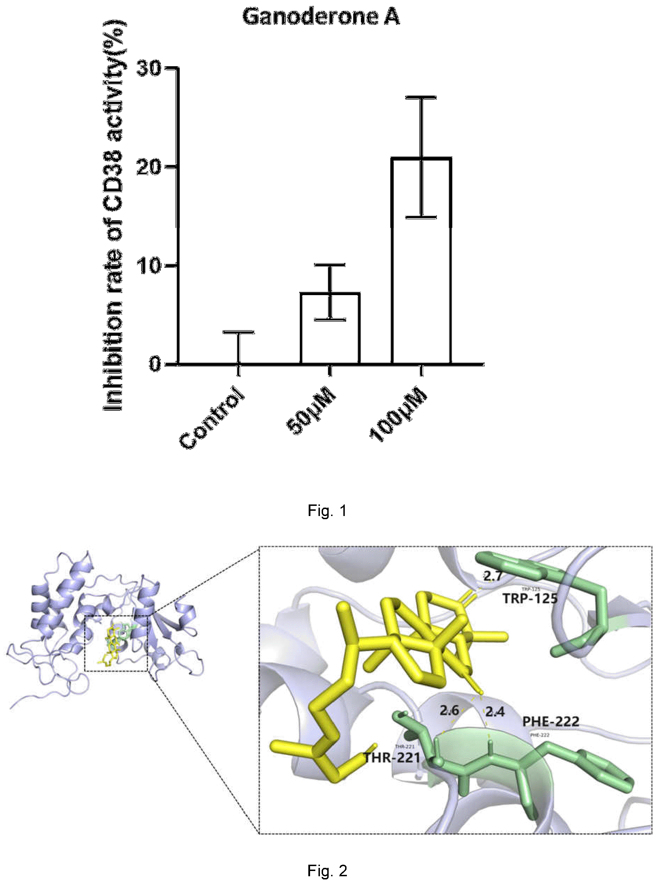

After incubation in a cell culture incubator for 20 minutes, a volume of 100 µL of supernatant was taken and transferred to a new 96-well plate.2. Detecting the residual amount of NAD⁺ and calculating the inhibition efficiency of CD38: adding an NAD⁺ reaction solution (which can react with NAD⁺ to make same appear), incubating at 37°C for 30 mi n. The microplate reader reads data and detects OD450, and calculates the inhibition efficiency of CD38 protein according to the OD450 value (inhibition rate= [(mock-control)-(mock-additional pore)]/(mock-control)). The results are shown in Fig. 1.

### Example 2: Experiments to Predict Direct Interactions of Ganoderone A and CD38

1. Manufacture of receptor, ligand manufacture: download 3D structure of CD38 protein in PDB database (PDB DOI: https: //www. rcsb. org/) preserved as pdb format, and oxygen and small molecules are removed from Pymol, and converted into pdbqt format in AutoDock; downloading the 3D structure of Ganoderone A in a PubChem database, storing same in a mol2 format, and importing same into an AutoDock and storing same in a pdbqt format.
2. Molecular docking of receptor and ligand: CD38 receptors prepared in S1 were introduced into AutoDock4 software, and the binding pocket of NAD⁺ to CD38 was used to calculate the lattice energy for the interface pocket. Then, a Ganoderone A ligand is introduced, and molecular docking is performed by means of AutoDock4 software, in which the number of times of docking is set as 100, and the conformational search precision is set as Medium. The docking result can be analyzed by clustering and binding, and a docking result with a lot of clusters and a low binding energy is selected and saved as a pdbqt format.
3. Visualization of docking result: The result stored in S2 is imported into a Protein-Ligand Interaction Profiler website, the interaction key is analyzed, and the interaction key is imported into Pymol software for visualization. The results are shown in Fig. 2.

### Example 3: Test for reduction of mitochondrial superoxide by Ganoderone A

1. Manufacture of cells: uniformly laying human dermal fibroblasts into a six-well plate, respectively processing an experimental group and a control group, and pre-setting blank tubes (i.e. not loading probes) to a cell density of 80-95%.
2. Digestion: cells in six-well plates were washed 1-2 times with PBS respectively, and PBS was discarded. The cells in the six-well plate were digested with pancreatin, transferred to a 1.5 ml LEP tube and labelled. 1200r, normal temperature, centrifuge for 5 min.
3. Manufacture of probe: an appropriate amount of MitoSO^{™} Red (5mM) is taken, the MitoSO^{™} Red is diluted according to the ratio of 1 mL of PBS (C0221A) added per 1 µl of MitoSO^{™} Red (5mM), and after mixing, the MitoSO^{™} Red is used as a MitoSO^{™} Red dyeing working solution. The medium was discarded, 1 mL of serum-free medium was added to the blank, 1 mL of prepared probe solution was added to the rest, and the cells were resuspended (taking care to avoid light).
4. Incubation: incubation at 37°C for 20 min in the dark. 1200r, centrifuge for 5 min at room temperature, and discard the supernatant. Wash with 1 mL cold PBS, 1200r, centrifuge for 5 mm at room temperature, discard the supernatant and repeat twice (note dark).
5. Data Acquisition: The cells were resuspended in 500 µL ice-cold PBS, transferred to flow cytometry tube, and subjected to flow cytometric acquisition.

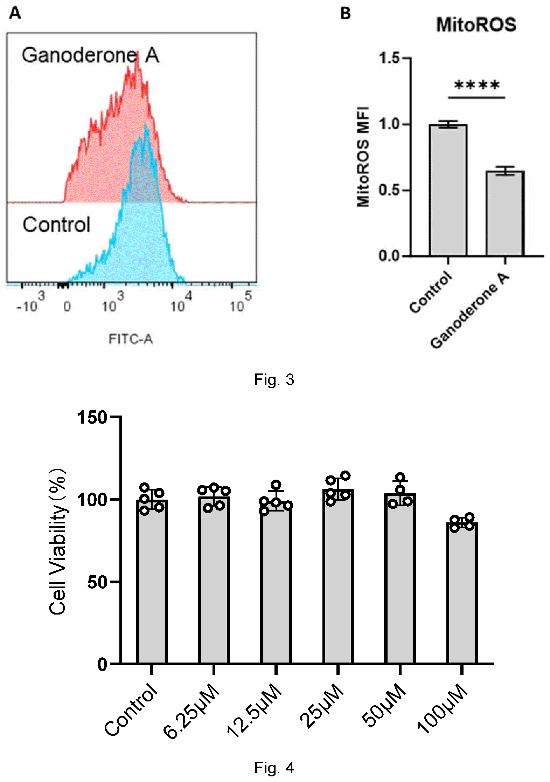

The results are shown in Fig. 3, in which A in Fig. 3 is a mitochondrial superoxide flow result graph, and B in Fig. 3 is a mean fluorescence intensity statistical graph of various groups of MitoROS.

### Example 4: BJ Safety Test of Ganoderone A on Skin Fibroblast

To determine the safety of Ganoderone A, we examined the effect of Ganoderone A on the viability of BJ cells by the CCK8 assay. Ganoderone A is dissolved in DMSO at 100 mM, and the Ganoderone A is added to the culture medium in a concentration gradient (6.25 µM, 12.5 µM, 25 µM, 50 µM and 100 µM). After culturing BJ cells for 24 h, 450 nm absorption of light is detected using a microplate reader, and cell viability is calculated, and the results are shown in Fig. 4. The results show that the cell viability of Ganoderone A is above 80% at the highest concentration, and has no obvious effect on cell viability.

From the above description, it can be seen that the above embodiments of the present application achieve the following technical effects: the present application finds that Ganoderone A acts as an inhibitor of CD38 and can inhibit the enzymatic activity of CD38, thereby promoting intracellular NAD⁺ levels and reducing mitochondrial superoxide activity and achieving an anti-aging effect. Furthermore, when using Ganoderone A as an inhibitor of CD38, Ganoderone A, due to its natural and mild properties, has no obvious effect on cell viability when acting on cells. Therefore, in the present application, when using Ganoderone A as an effective component of a CD38 inhibitor, effective anti-fatigue and biological safety can be taken into account, and the manufacturing costs are lower than those of the CD38 inhibitor in the prior art, which facilitates application and promotion, and facilitates research and development in the field of anti-aging.

## Claims

1. The Ganoderone A for use as a CD38 inhibitor.

2. The Ganoderone A for use as a CD38 inhibitor according to claim 1, wherein a concentration of the Ganoderone A in the CD38 inhibitor is 50-100 µM.

3. The Ganoderone A for use in a method of manufacturing an anti-aging product, wherein the Ganoderone A inhibits CD38 for anti-aging.

4. The Ganoderone A for use in the method of manufacturing an anti-aging product according to claim 3, wherein the product comprises a cosmetic product, a skin care product, a healthcare product, or a pharmaceutical product.

5. The Ganoderone A for use in the method of manufacturing an anti-aging product according to claim 3, wherein the anti-aging comprises: increasing the level of NAD⁺ in the cell and/or decreasing the activity of superoxide in the mitochondria of the cell by inhibiting CD38 protein.

6. The Ganoderone A for use in the method of manufacturing an anti-aging product according to claim 3, wherein a concentration of the Ganoderone A in the product is 50-100µM.

7. The Ganoderone A for use in the method of manufacturing an anti-aging product according to claim 3, wherein the product further comprises an auxiliary and/or a carrier.

8. The Ganoderone A for use in the method of manufacturing an anti-aging product according to claim 7, the auxiliary comprises an antioxidant, a humectant, a collagen promoter, a solvent, a stabilizer, a diluent, a binder, an antisticking agent, a cosolvent, a film coating material, a slow-release matrix material, a solubilizer, an emulsifier, a preservative, an isotonic modulator or a colorant.

9. The Ganoderone A for use in a method of manufacturing a product that increase the level of NAD⁺ in cells or decrease the activity of superoxide in mitochondria of cells; wherein the Ganoderone A inhibits CD38 for increasing the level of NAD⁺ in cells or decreasing the activity of superoxide in mitochondria of cells.

10. The Ganoderone A for use in the method of manufacturing a product that increase the level of NAD⁺ in cells or decrease the activity of superoxide in mitochondria of cells according to claim 9, wherein a concentration of the Ganoderone A in the product is 50-100µM.

11. The Ganoderone A for use in the method of manufacturing a product that increase the level of NAD⁺ in cells or decrease the activity of superoxide in mitochondria of cells according to claim 9, wherein the product further comprises an auxiliary and/or a carrier.

12. The Ganoderone A for use in the method of manufacturing a product that increase the level of NAD⁺ in cells or decrease the activity of superoxide in mitochondria of cells according to claim **11,** the auxiliary comprises an antioxidant, a humectant, a collagen promoter, a solvent, a stabilizer, a diluent, a binder, an antisticking agent, a cosolvent, a film coating material, a slow-release matrix material, a solubilizer, an emulsifier, a preservative, an isotonic modulator or a colorant.
